# EUROPÄISCHE PATENTSCHRIFT

(11) **EP 1 202 715 B1**
(45) Veröffentlichungstag und Bekanntmachung des Hinweises auf die Patenterteilung: **12.09.2007**
(21) Anmeldenummer: 00949311.5
(22) Anmeldetag: 12.07.2000
(51) Int. Cl.: A61K 9/20

(54) **SCHNELLFREISETZENDE EXTRUDATE UND VERFAHREN ZU IHRER HERSTELLUNG SOWIE DARAUS ERHÄLTLICHE ZUBEREITUNGEN**
QUICK-RELEASE EXTRUDATES, METHOD FOR PREPARING THE SAME AND COMPOSITIONS OBTAINED FROM SAID EXTRUDATES
EXTRUDATS A LIBERATION RAPIDE, LEUR PROCEDE DE PRODUCTION ET PREPARATIONS POUVANT ETRE OBTENUES PARTIR DESDITS EXTRUDATS

(30) Priorität: 23.07.1999 DE 19934610
(43) Veröffentlichungstag der Anmeldung: 08.05.2002
(73) Patentinhaber: Abbott GmbH & Co. KG, 65205 Wiesbaden (DE)
(72) Erfinder: KANIKANTI, Venkata-Rangarao, D-51381 Leverkusen (DE); SDEBIK, Jürgen, D-40476 Düsseldorf (DE)
(74) Vertreter: Thalhammer, Wolfgang
(86) Internationale Anmeldenummer: PCT/EP2000/006584
(87) Internationale Veröffentlichungsnummer: WO 2001/007015

(56) Entgegenhaltungen:
- EP-A- 0 596 203
- EP-A- 0 864 326
- WO-A-93/18755
- WO-A-96/25149
- WO-A-96/25151
- DE-A- 19 536 387
- US-A- 4 317 447

## Beschreibung

Die vorliegende Erfindung betrifft eine pharmazeutische Zubereitung in Single-Unit-Dosisform, umfassend ein stabiles, schnellfreisetzendes Extrudat in zerkleinerter Form und ein Sprengmittel, wobei das Extrudat Hydroxypropylcellulose niedriger Viskosität mit einer mittleren Molekulargewicht von 30.000 bis 100.000 und einem molaren Substitutionsgrad von mindestens 3 in einer Menge von mindestens 50 Gew.-% bezogen auf das Wirfstoff-Polymergemisch und mindestens einen pharmazeutischen Wirkstoff enthält.

Die genaue Steuerung der Wirkstofffreisetzung aus Zubereitungen ist von großer pharmazeutischer Bedeutung. Neben Retardzubereitungen und solchen mit kontrollierter Freisetzung werden schnellfreisetzende Zubereitungen eingesetzt, die eine schnelle Anflutung des Wirkstoffes im Kreislauf bei akutem Krankheitsbild gewährleisten.

Bei der Herstellung entsprechender Zubereitungen werden häufig Extrusionsverfahren eingesetzt. Das Prinzip der Schmelzextrusion ist bereits seit langem bekannt (Beckmann 1964). Beim Extrusionsverfahren werden Wirkstoffe und Polymer entweder gleichzeitig, ohne vorheriges Mischen, oder als Mischung, nach vorherigem Mischen, in einem Extruder gefördert, welcher derart erhitzt wurde, dass die Mischung extrudierbar wird und der Wirkstoff nicht abgebaut wird.

Es ist hierbei im Gegensatz zu herkömmlichen Copräzipitat-Methoden der Einsatz von Lösungsmitteln überflüssig: dies ist insbesondere wichtig, da der Einsatz von Lösungsmitteln neben wirtschaftlichen Aspekten spezielle technische Fragestellungen aufwirft, wie Explosionsschutz der Räume und Geräte.

Das Dokument DE-A-19536387 betrifft ein Verfahren zur Herstellung von vitaminhaltigen Arzneiformen durch Extrusion einer vitaminhaltige Polymerschmelze, die Hydroxypropylcellulose enthält.

Die Herstellung von Zubereitungen mit schneller Wirkstofffreisetzung durch Schmelzextrusion von Hydroxypropylcellulose (HPC) mit einem Molekulargewicht von 60.000 bis 200.000 wird in der Anmeldung WO 96/25149 beschrieben. Bei diesen Zubereitungen beträgt der Anteil an Hydroxypropylcellulose 10 bis 30, bevorzugt 20 bis 30 Gew.-%.

Solche niedrigen Anteile an Polymer sind aus pharmazeutischer Sicht nachteilig, da die resultierende Zubereitung insbesondere bei der Verwendung hochpotenter Wirkstoffe sehr klein und damit für den Patienten schwer handhabbar wird, was die sog. Patienten-Compliance erheblich verschlechtert. Ein Verstrecken der Zubereitung mit Füllstoffen wiederum erhöht die Anzahl der verwendeten Inhaltsstoffe und verkompliziert somit das Verfahren. Dies ist nachteilig u.a. im Hinblick auf die Kompatibilität der Füllstoffe mit dem Wirkstoff insbesondere bei erhöhter Temperatur während der Extrusion.

Ein weiterer Nachteil ist die schwerer einzuhaltende "Content Uniformität", d.h. die Einheitlichkeit der Zubereitungen im Hinblick auf die Wirkstoffmengen.

Eine Aufgabe der vorliegenden Erfindung ist darin zu sehen, schnellfreisetzende, stabile Extrudate mit guter Content Uniformität und Handhabbarkeit für alle Arten von Wirkstoffen, insbesondere für schwerlösliche Wirkstoffe zur Verfügung zu stellen.

Überraschend wurde nun gefunden, dass mit dem speziell ausgewählten Polymer Hydroxypropylcellulose niedriger Viskosität mit einem mittleren Molekulargewicht von 30.000 bis 100.000 und einem molaren Substitutionsgrad von mindestens 3 in einer Menge von mindestens 50 Gew.-%, bevorzugt 66 Gew.-% bezogen auf das Wirkstoff-Polymergemisch in Verbindung mit amorphen Wirkstoffen transparente Schmelzextrudate mit der gewünschten schnellen Freisetzung und hoher Stabilität in einfacher Weise erhältlich sind.

Die erfindungsgemäßen Schmelzextrudate enthalten Hydroxypropylcellulose niedriger Viskosität mit einem mittleren Molekulargewicht von 30.000 bis 100.000 und einem molaren Substitutionsgrad von mindestens 3 in einer Menge von mindestens 50 Gew.-%, bevorzugt 66 Gew.-% bezogen auf das Wirkstoff-Polymergemisch, einen oder mehrere Wirkstoffe und gegebenenfalls Hilfsstoffe.

Bevorzugt sind Schmelzextrudate, die Hydroxypropylcellulose in einer Menge von mindestens 66 Gew.-% bezogen auf das Wirkstoff-Polymergemisch enthalten.

Die Hydroxypropylcellulose weist bevorzugterweise ein mittleres Molekulargewicht von 55.000 bis 70.000 auf.

Molarer Substitutionsgrad bezieht sich auf die durchschnittliche Anzahl von Molen Propylenoxid, die pro Glucoseeinheit der Cellulose umgesetzt sind.

Gegenstand der vorliegenden Anmeldung sind auch Zubereitungen, die unter Verwendung der erfindungsgemäßen Extrudate hergestellt werden. Unter schnellfreisetzenden Extrudaten oder Zubereitungen werden im Rahmen der vorliegenden Erfindung solche verstanden, die den oder die Wirkstoffe gemäß USP XXII-Paddle-Methode innerhalb von 60 min zu 80 % freisetzen. Die erfindungsgemäßen Extrudate oder Zubereitungen weisen eine sehr schnelle Freisetzung auf und zeigen eine signifikante Übersättigung. Sie sind deshalb besonders zum schnellen Erreichen hoher Plasmawirkstoffkonzentrationen sehr geeignet. Die schnelle Freisetzung kann durch Variation der Herstellungsparameter je nach Bedarf kontrolliert werden. Die Wirkstofffreisetzung wird z.B. beeinflusst durch die Wirkstoffkonzentration im Endprodukt oder durch Verfahrensparameter der Extrusion, wie die Schneckengeometrie, die Extrusionsrate, die Extrusionstemperatur, der Durchmesser und die Oberfläche des Extrudats usw. Insbesondere wird die Freisetzungsrate beeinflusst durch die Partikelgröße der Extrudate. Bei Single-Unit-Dosisformen, wie z.B. Tabletten wird die Freisetzung insbesondere durch den Anteil an Sprengmittel in der fertigen Formulierung beeinflusst.

Eine schnelle, aber zeitlich unterschiedliche Freisetzung des Wirkstoffs bei oraler Applikation kann erreicht werden, indem die Extrudate oder Zubereitungen derart lackiert werden, dass sie den Wirkstoff erst in einer bestimmten Region des Magen-Darm-Trakts freisetzen, z.B. mit Eudragit L oder S. Derartige Lacke werden z.B. spezifisch bei bestimmten pH-Werten aufgelöst und ermöglichen die Freisetzung am optimalen Ort. Auf diese Weise können z.B. säureempfindliche Wirkstoffe bis zum Erreichen des entsprechenden Resorptionsfensters geschützt werden (siehe dazu Chang, R., Pharmaceutical Technology, October 1990).

Während der Schmelzextrusion oder bei der weiteren Verarbeitung, z.B. Tablettierung, können auch weitere übliche Hilfsstoffe verwendet werden, die bei der Herstellung von Zubereitungen in der Pharmazie üblich und aus der Literatur bekannt sind, wie z.B. Magnesiumstearat oder Geschmackskorrigentien. Keiner dieser Hilfsstoffe ist jedoch notwendig, um die gewünschte schnelle Freisetzung des Arzneistoffs wesentlich zu erreichen.

In einer bevorzugten Ausführungsform wird das Extrudat oder die Zubereitung oral appliziert. Dazu wird in einer Ausführungsform das erfindungsgemäße Extrudat zerkleinert, z.B. gemahlen zu einem durchschnittlichen Partikeldurchmesser von weniger als 0.5 mm und in Gelatinekapseln oder als Sachets abgefüllt.

In einer weiteren Ausführungsform wird das erfindungsgemäße Extrudat in zerkleinerter Form mit einem Sprengmittel und ggf. weiteren Hilfsstoffen gemischt und zu einer Single-Unit-Dosisform verarbeitet. Sprengmittel sind Stoffe, die in wässriger Lösung für ein schnelles Zerfallen der Single-Unit-Dosisform sorgen, wie z.B. quervernetztes Polyvinylpyrrolidon (PVPP). Hierbei werden bevorzugterweise hohe Anteile an Sprengmittel verwendet, d.h. mehr als 0,1 Gewichtsteil Sprengmittel auf 1 Teil Extrudat (Wirkstoff und HPC).

Unter Single-Unit-Dosisformen werden solche Zubereitungen verstanden, die als Einzelgabe verabreicht werden, z.B. Tabletten, Dragees oder Kapseln.

Es ist auf diese Weise weiterhin möglich, Kombinationspräparate herzustellen, die in einer Single-Unit-Dosisform Anteile verschiedener wirkstoffhaltiger Extrudate enthalten: So können verschiedene Wirkstoffe, aber auch verschiedene Extrudate mit unterschiedlichem Freisetzungsprofil verwendet werden, um Wirkstoffgehalte im Kreislauf zeitlich genau zu steuern.

Bei dem zu verwendenden Wirkstoffkann es sich um beliebige Arzneistoffe handeln, wie z.B. Antiinfektiva, Kreislaufmittel, Antimykotika, Antidepressiva, Antidementika, Antiepileptika, Antiphlogistika, Analgetika, Antiasthmatika, Antithrombotika, Antitumormittel, Antimalariamittel, nichtsteroidale entzündungshemmende Mittel (NSAID), Diuretika, Antiarrhythmika, blutzuckersenkende Mittel, ACE-Hemmer, Sedativa, Decongestiva, Antihistaminika oder Lipidsenker. Weiterhin kommen als Wirkstoffe auch Pflanzenschutzmittel oder Tierarzneimittel in Betracht. Für die Zwecke der vorliegenden Erfindung werden nur diejenigen Arzneistoffe eingearbeitet, die sich unter den Temperaturen und Verarbeitungsbedingungen nicht zersetzen. Die zu verabreichende Wirkstoffmenge pro Dosiseinheit kann je nach Art des Arzneistoffs und der Freisetzungsrate innerhalb weiter Grenzen variiert werden. Bevorzugt werden schwerlösliche Wirkstoffe, d.h. solche, die eine Löslichkeit von weniger als 1 g/100-1000 g Lösungsmittel aufweisen. Es ist weiterhin möglich, Mischungen von Arzneistoffen einzusetzen.

Gegenstand der vorliegenden Erfindung ist weiterhin ein Verfahren zur Fertigung der erfindungsgemäßen Extrudate und Zubereitungen. Bei diesem einfachen Verfahren benötigt man nur einen herkömmlichen Extruder und kommt ohne komplizierte Fertigungsmethoden oder Lösungsmittel und Weichmacher aus.

Beim vorliegenden Verfahren stellt man eine Mischung aus mindestens einem Wirkstoff und Hydroxypropylcellulose niedriger Viskosität in einer Menge von mindestens 50 Gew.-%, insbesondere mindestens 66 Gew.-% bezogen auf das Wirkstoff-Polymergemisch und gegebenenfalls weiteren üblichen pharmazeutischen Hilfsstoffen her und leitet die Mischung durch einen Extruder, der an der Stelle des Produkteintritts eine Temperatur von 20 bis 40°C und an der oder den Austrittsdüsen eine Temperatur von ≤ 225 °C aufweist, wobei die Austrittsdüsen einen Durchmesser von 0,5 bis 5 mm, vorzugsweise von 1 bis 3 mm aufweisen, und die extrudierten Stränge nach ihrem Austritt zerkleinert. Die Verweilzeit der Mischung im Extruder ist dabei von verfahrenstechnischen Aspekten abhängig und kann in breitem Maße variieren. Sie liegt i.A. unter 3 min, bevorzugterweise unter 1 min.

Die Mischung der Bestandteile kann vor dem Eintritt in den Extruder oder in diesem erfolgen. Die Vormischung, also die Mischung vor dem Eintritt, ist u.a. aus Gründen der o.g. Content-Uniformität bevorzugt. Ebenso ist ein kontinuierlicher Betrieb des Extruders bevorzugt.

Überraschenderweise wurde gefunden, dass bei dem erfindungsgemäßen Verfahren auch bei hohen Anteilen an Hydroxypropylcellulose ohne den Einsatz von Weichmachern oder Lösungsmitteln extrudiert werden kann. Dies war für den Fachmann nicht zu erwarten, da mit einem steigendem Anteil an Hydroxypropylcellulose mit einem herkömmlichen Extruder im allgemeinen eine verschlechterte Extrudierbarkeit einhergeht.

Die Viskosität wird mit einem Rotationsviskometer auf bekannte Weise bei 20°C anhand einer 2%-igen wässrigen Lösung bestimmt; die niedrigviskosen Cellulosen der vorliegenden Erfindung weisen Viskositäten von weniger als 400 mPa·s auf.

Die Erfindung wird anhand der nachfolgenden Beispiele näher erläutert.

### Ausführungsbeispiele

### Messung der Wirkstofffreisetzung nach USP XXII Paddle-Methode

Die Freisetzung der o.g. Wirkstoffe aus den Tabletten oder Gelatinekapseln wird gemäß USP XXII Paddle-Methode gemessen. Rührerdrehzahl 75 upm, Freisetzungsmedium 0,1 N HCl (0,9 L) mit kleinen Mengen von Lösungsvermittlern (z.B.: ≤0,5 (w/v) Natriumlaurylsulfat), Temperatur 37°C. Alle erfindungsgemäßen Beispiele außer Vergleichsbeispiel A setzen ca. 80% Wirkstoff innerhalb 60 min frei.

### Beispiel 1

1 Gewichtsteil Nifedipin wird mit 2 Gewichtsteilen niedrigviskosem HPC (mittleres MG 55000 - 70000) gemischt. Die Mischung wird auf einem Doppelschneckenextruder mit einer Austrittsdüse mit einem Durchmesser von 2 mm verarbeitet. Das Material wird bei einer Düsentemperatur von 170°C extrudiert. Die transparenten Extrudatstränge werden zerkleinert und die Fraktion (< 125µm ; 90 Gewichtsteile) mit 119 Gewichtsteilen quervernetztem Polyvinylpyrrolidone (PVPP) und anschließend mit Magnesiumstearat (1 Gewichtsteil) gemischt und tablettiert ( 30 mg Nifedipin pro Tablette).

### Beispiel 2

1 Gewichtsteil Nifedipin wird mit 2,5 Gewichtsteilen niedrigviskosem HPC (mittleres MG 55000 - 70000) gemischt. Die Mischung wird auf einem Doppelschneckenextruder mit einer Austrittsdüse mit einem Durchmesser von 2 mm verarbeitet. Das Material wird bei einer Düsentemperatur von 170°C extrudiert. Die transparenten Extrudatstränge werden zerkleinert und die Fraktion (< 125 µm; 105 Gewichtsteile) mit 135 Gewichtsteilen quervernetztem Polyvinylpyrrolidone (PVPP) und anschließend mit Magnesiumstearat (1,2 Gewichtsteile) gemischt und tablettiert (30 mg Nifedipin pro Tablette).

### Beispiel 3

Analog Beispiel 1, jedoch beträgt die Düsentemperatur 160°C.

### Beispiel 4

Analog Beispiel 1, jedoch wird als Arzneistoff Nimodipin verwendet; die Düsentemperatur beträgt 120°C.

### Beispiel 5

1 Gewichtsteil 2-Cyclopentyl-2-[4-(2,4-dimethyl-pyrido[2,3-*b*]indol-9-ylmethyl)-phenyl]-*N*-(2-hydroxy-1-phenyl-ethyl)-acetamid wird mit 2 Gewichtsteilen niedrigviskosem HPC (mittleres MG 55000 - 70000) gemischt. Die Mischung wird auf einem Doppelschneckenextruder mit einer Austrittsdüse mit einem Durchmesser von 2 mm verarbeitet. Das Material wird bei einer Düsentemperatur von 220°C extrudiert. Die transparenten Extrudatstränge werden zerkleinert und die Fraktion (< 125µm; 240 Gewichtsteile) mit 118,8 Gewichtsteilen quervernetztem Polyvinylpyrrolidone (PVPP) und anschließend mit Magnesiumstearat (1,2 Gewichtsteile) gemischt und tablettiert (80 mg (2*S*)-2-Cyclopentyl-2-[4-(2,4-dimethyl-pyrido[2,3-*b*]indol-9-ylmethyl)-phenyl]-*N*-(1*R*)-2-hydroxy-1-phenyl-ethyl)-acetamid pro Tablette).

### Beispiel 6

Analog Beispiel 1, jedoch wird die Mischung in Gelatinekapseln abgefüllt.

### Beispiel 7

Analog Beispiel 4, jedoch wird die Mischung in Gelatinekapseln abgefüllt.

### Vergleichsbeispiel A

Analog Beispiel 5, jedoch werden 2 Gewichtsteile des Arzneistoffs 2-Cyclopentyl-2-[4-(2,4-dimethyl-pyrido[2,3-*b*]indol-9-ylmethyl)-phenyl]-*N*-(2-hydroxy-1-phenylethyl)-acetamid mit 1 Gewichtsteil niedrigviskosem HPC (mittleres MG 55000 - 70000) gemischt . Die Mischung wird auf einem Doppelschneckenextruder mit einer Austrittsdüse mit einem Durchmesser von 2 mm verarbeitet. Das Material wird bei einer Düsentemperatur von 220°C extrudiert. Die Extrudatstränge werden zerkleinert und die Fraktion (< 125µm; 240 Gewichtsteile) wird mit 118,8 Gewichtsteilen quervernetztem Polyvinylpyrrolidone (PVPP) und anschließend mit Magnesiumstearat (1,2 Gewichtsteile) gemischt und tablettiert (80mg (2*S*)-2-Cyclopentyl-2-[4-(2,4-dimethyl-pyrido[2,3-*b*]indol-9-ylmethyl)-phenyl]-*N*-(1*R*)-2-hydroxy-1-phenyl-ethyl)acetamid pro Tablette).

## Patentansprüche

1. Pharmazeutische Zubereitung in Single-Unit-Dosisform, umfassend ein stabiles, schnellfreisetzendes Extrudat in zerkleinerter Form und ein Sprengmittel, wobei das Extrudat Hydroxypropylcellulose niedriger Viskosität mit einem mittleren Molekulargewicht von 30.000 bis 100.000 und einem molaren Substitutionsgrad von mindestens 3 in einer Menge von mindestens 50 Gew.-%, bezogen auf das Wirkstoff-Polymergemisch und mindestens einen pharmazeutischen Wirkstoff enthält.

2. Pharmazeutische Zubereitung gemäß Anspruch 1, **dadurch gekennzeichnet, dass** das Extrudat Hydroxypropylcellulose in einer Menge von mindestens 66 Gew.-%, bezogen auf das Wirkstoff-Polymergemisch enthält.

3. Pharmazeutische Zubereitung gemäß Anspruch 1 oder 2, **dadurch gekennzeichnet, dass** das Extrudat Hydroxypropylcellulose mit einem mittleren Molekulargewicht von 55.000 bis 70.000 enthält.

4. Pharmazeutische Zubereitung gemäß einem der Ansprüche 1 bis 3, **dadurch gekennzeichnet, dass** sie mehr als 0,1 Gewichtsteil Sprengmittel auf 1 Teil Extrudat enthält.

5. Pharmazeutische Zubereitung gemäß einem der Ansprüche 1 bis 4, **dadurch gekennzeichnet, dass** das Sprengmittel quervernetztes Polyvinylpyrrolidon ist.

6. Pharmazeutische Zubereitung gemäß einem der Ansprüche 1 bis 5, **dadurch gekennzeichnet, dass** die Zubereitung eine Tablette ist.

7. Pharmazeutische Zubereitung gemäß einem der Ansprüche 1 bis 5, **dadurch gekennzeichnet, dass** die Zubereitung ein Sachet ist.

8. Pharmazeutische Zubereitung gemäß einem der Ansprüche 1 bis 5, **dadurch gekennzeichnet, dass** die Zubereitung eine Kapsel ist.

9. Lösungsmittelfreies Verfahren zur Herstellung einer pharmazeutischen Zubereitung gemäß Anspruch 1, **dadurch gekennzeichnet, dass** eine Mischung aus mindestens einem Wirkstoff und Hydroxypropylcellulose niedriger Viskosität in einer Menge von mindestens 50 Gew.-% bezogen auf das Wirkstoff-Polymergemisch durch einen Extruder geleitet wird, der an der Stelle des Produkteintritts eine Temperatur von 25 bis 40 °C und an der Austrittsdüse eine Temperatur von ≤ 225 °C aufweist, wobei die Austrittsdüse(n) einen Durchmesser von 0,5 bis 5 mm aufweist(/en), und man die extrudierten Stränge nach ihrem Austritt zerkleinert und mit einem Sprengmittel mischt.

10. Verfahren gemäß Anspruch 9, **dadurch gekennzeichnet, dass** die Austrittsdüse(n) einen Durchmesser von 1 bis 3 mm aufweist(/en).

## Claims

1. Pharmaceutical preparation in the form of single dosage units, comprising a stable, quick-release extrudate in comminuted form and a disintegrant, the extrudate containing low viscosity hydroxypropylcellulose with a mean molecular weight of 30,000 to 100,000 and a molar substitution level of at least 3, in an amount of at least 50 wt.%, based on the active substance/ polymer mixture, and at least one pharmaceutical active substance.

2. Pharmaceutical preparation according to claim 1, **characterised in that** the extrudate contains hydroxypropylcellulose in an amount of at least 66 wt.%, based on the active substance/ polymer mixture.

3. Pharmaceutical preparation according to claim 1 or 2, **characterised in that** the extrudate contains hydroxypropylcellulose with a mean molecular weight of 55,000 to 70,000.

4. Pharmaceutical preparation according to one of claims 1 to 3, **characterised in that** it contains more than 0.1 part by weight of disintegrant to 1 part of extrudate.

5. Pharmaceutical preparation according to one of claims 1 to 4, **characterised in that** the disintegrant is cross-linked polyvinylpyrrolidone.

6. Pharmaceutical preparation according to one of claims 1 to 5, **characterised in that** the preparation is a tablet.

7. Pharmaceutical preparation according to one of claims 1 to 5, **characterised in that** the preparation is a sachet.

8. Pharmaceutical preparation according to one of claims 1 to 5, **characterised in that** the preparation is a capsule.

9. Solvent-free method of preparing a pharmaceutical preparation according to claim 1, **characterised in that** a mixture of at least one active substance and low viscosity hydroxypropylcellulose in an amount of at least 50 wt.% based on the active substance/polymer mixture is passed through an extruder which has a temperature of 25 to 40°C at the point of entry of the product and a temperature of ≤ 225°C at the exit nozzle, the exit nozzle(s) having a diameter of 0.5 to 5 mm, and after emerging the extruded strips are comminuted and mixed with a disintegrant.

10. Method according to claim 9, **characterised in that** the exit nozzle(s) has (have) a diameter of 1 to 3 mm.

## Revendications

1. Préparation pharmaceutique sous forme de dose unitaire, comprenant un extrudat stable à libération rapide sous forme broyée et un agent désintégrant, dans laquelle l'extrudat contient une hydroxypropylcellulose de faible viscosité, ayant une masse molaire moyenne de 30 000 à 100 000 et un degré de substitution molaire d'au moins 3, en une quantité d'au moins 50 % en masse par rapport au mélange de substance active et de polymère, et au moins une substance active pharmaceutique.

2. Préparation pharmaceutique selon la revendication 1, **caractérisée en ce que** l'extrudat contient de l'hydroxypropylcellulose en une quantité d'au moins 66 % en masse par rapport au mélange de substance active et de polymère.

3. Préparation pharmaceutique selon la revendication 1 ou 2, **caractérisée en ce que** l'extrudat contient une hydroxypropylcellulose ayant une masse molaire moyenne de 55 000 à 70 000.

4. Préparation pharmaceutique selon l'une des revendications 1 à 3, **caractérisée en ce qu'**elle contient plus de 0,1 partie en masse d'agent désintégrant pour 1 partie d'extrudat.

5. Préparation pharmaceutique selon l'une des revendications 1 à 4, **caractérisée en ce que** l'agent désintégrant est une polyvinylpyrrolidone réticulée.

6. Préparation pharmaceutique selon l'une des revendications 1 à 5, **caractérisée en ce que** la préparation est un comprimé.

7. Préparation pharmaceutique selon l'une des revendications 1 à 5, **caractérisée en ce que** la préparation est un sachet.

8. Préparation pharmaceutique selon l'une des revendications 1 à 5, **caractérisée en ce que** la préparation est une capsule.

9. Procédé sans solvant pour la production d'une préparation pharmaceutique selon la revendication 1, **caractérisé en ce que** l'on fait passer un mélange d'au moins une substance active et d'hydroxypropylcellulose de faible viscosité en une quantité d'au moins 50 % en masse par rapport au mélange de substance active et de polymère dans une extrudeuse qui présente au site d'introduction du produit une température de 25 à 40°C et à la filière de sortie une température ≤ 225°C, la ou les filières de sortie ayant un diamètre de 0,5 à 5 mm, et on broie les joncs extrudés après leur sortie et on les mélange avec un agent désintégrant.

10. Procédé selon la revendication 9, **caractérisé en ce que** la ou les filières de sortie ont un diamètre de 1 à 3 mm.
